# EUROPEAN PATENT APPLICATION

(11) **EP 0 527 307 A2**
(43) Date of publication of application: **17.02.1993**
(21) Application number: 92109982.6
(22) Date of filing: 13.06.1992
(51) Int. Cl.: G01N 33/00, G01W 1/00, G08G 1/00

(54) **Integrated system for the detection and measurement of the urban traffic pollutants and method thereof**

(30) Priority: 14.06.1991 IT RM910428
(71) Applicant: ALENIA AERITALIA & SELENIA S.P.A., I-00131 Roma (IT)
(72) Inventor: Barale, Giancarlo, I-00138 Roma (IT); Nussio, Fabio, I-00154 Roma (IT)
(74) Representative: Gustorf, Gerhard, Dipl.-Ing.

(57) **Abstract**

Integrated system for the detection and management of the urban environment pollution, consisting of:
-a fixed measurement network of the atmospheric pollutants (1); -meteorologic network (4);- automatic traffic detector network (3); - mobile laboratories which check air quality (2); -Operations centre for storage, management and processing of the data produced by the networks above(7); -information and forecast distribution system (5) (6).
The system and methods thereof belong to the field of electronic systems, however,due to its field of application, it pertains to the field of environment and public health safeguarding systems, and it finds its best application in urban, regional etc areas.

## Description

This invention regards a method for the detection, extrapolation and management of the urban traffic pollution, with particular emphasis on the pollution by Carbon Monoxide (CO) which is, as well known, due to vehicles in 99% of cases.

The extrapolation is applied to input data from the fixed pollutant measurement network, from the mobile labs and from the meteo networks, as well as from the traffic detector network so as to result in an evaluation of pollution levels in all points of interest to the area.

Management is applied to the short term weather forecast provided by the weather offices, so that by forecast and considerations on the traffic (expected public works or civil assemblies or other), a pollution-critical situation can be avoided. Or it can provide people with tools which can impede the arising of such situations. As an example, for public works , alternative access routes could be indicated to avoid queuing, which always bears increased pollution.

Till now, detection system have provided point measurements, with doubtful effectiveness in terms of extrapolation based upon statistics.

The inconveniences presented by these systems are due to the fact that these measurements are necessarily partial, an unbearable fact for an urban environment, where the microclimatic conditions differ widely from one point to the other of a town.

The system presented herein and for which a patent application is made, provides advantages in terms of planning and design of developments of the monitoring network and the optimum choice of measurement sites as well as the space extrapolation of pollution levels: all this is obtained through the measurements performed by the monitoring networks of the system.
The system and procedures thereof, provide short term forecast of the level of pollutants.

Moreover, the same structure could also be adopted to detect and measure noise pollution as well as other pollutants.

The procedure, due to the detection networks, can compare measured values with those calculated in real time, resulting in an autocorrelation, and therefore in the correction, so as to acquire an increasingly more accurate knowledge of the system, evolving towards an expert system dedicated to the solving of the pollution problem .

The combined system and its procedures, which are the subject of this patent application, can be applied preferably to urban areas subject to pollution and wherever decisions have to be taken with the help of tools which improve the knowledge of problems such as that of pollution.

Due to their nature, the system and the procedures adopted belong to the field of electronics; due to their scope, they belong also to the field of procedures and systems for the safeguard of the environment and of public health.

The invention will now be described with reference to the tables of figures enclosed, with the intent to illustrate its characteristics without limiting them to this description.

The part indicated as(7) is made up of two main parts: a) is the data processing interface, which collects, presents and stores the data coming from the monitoring network, as well as providing for its analysis; b) is the modelling part.
This block 7 is a system which processes the entire procedure and could well be represented by a graphic workstation, such as that of the MARA/XR series manufactured by the applicant.

Figure 1 is a schematic of the procedure subject of this patent application.

Here we can see:
1 fixed network for the measurement of pollutants;
2 mobile laboratories;
3 traffic detector system;
4 weather network;
5&6 data diffusion and prediction system;
7 Operations centre.

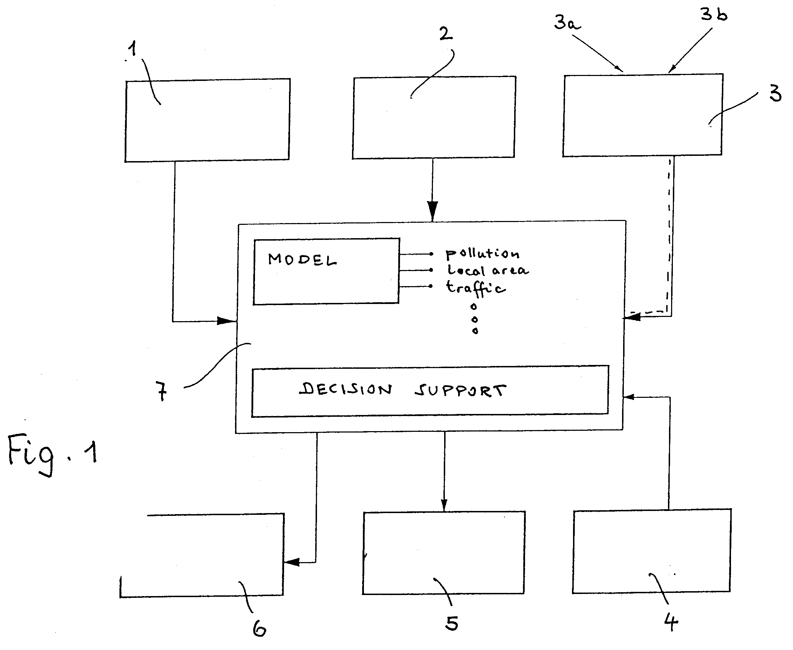

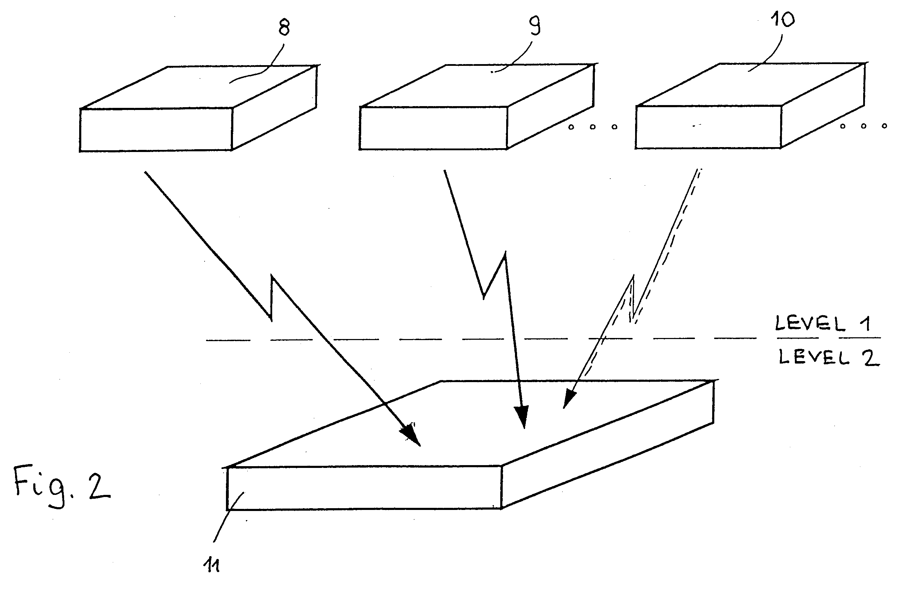

Figure 2 is the schematic representation of the modelling network.
Here we can see:
8 CO pollution mode
9 noise pollution model;
10 traffic update model;
11 local area model.

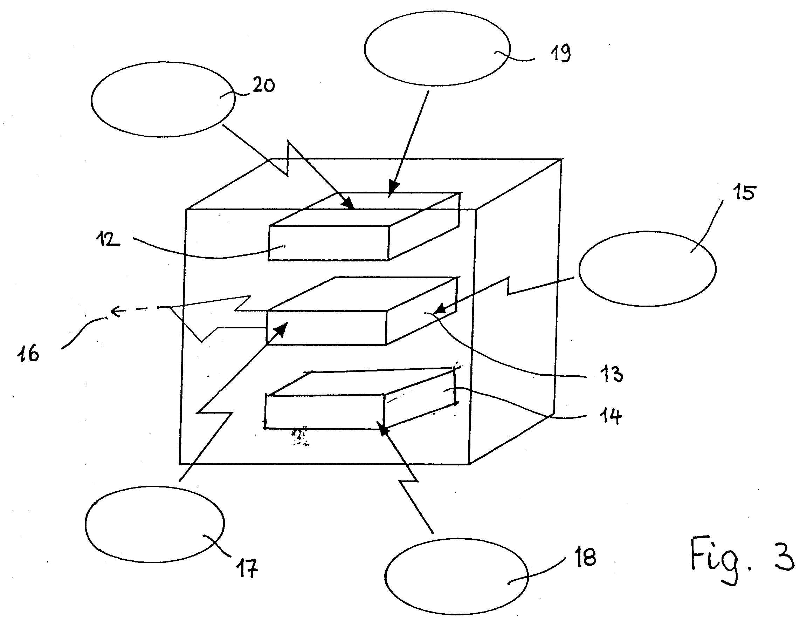

Figure 3 shows the detail of the model for the Carbon Monoxide pollution and it shows:
12 traffic model;
13 thermodynamic model;
14 calibration and update model;
15 weather forecast;
16 environmental conditions;
17 weather monitor network;
18 pollutant monitor network;
19 traffic statistic data;
20 traffic monitor network.

As this text explains, the original feature of this invention lies in the system and in its procedures. Data is not present in any of the components of the system, but become available at the end of the procedure.
The fixed measurement network (1) for the atmospheric pollutants consists of a set of stations equipped with the right type of sensors to detect the main atmospheric pollutants such as Sulphur Dioxide, SO2, Carbon Monoxide CO, Nitrogen oxides NOx, Ozone O3, Hydrocarbons HC and Suspended Total Dust TSD and to monitor the noise pollution, provided with communication lines to send data to the operations centre.

The weather network (4) consists of a set of stations which contain the main weather sensors such as wind direction and speed sensors.pressure, sun rate, pluviometer,rain acidity, all connected to the operations centre for data transmission.

The Automatic Traffic detector network (3) consists of fixed stations containing the sensors required to measure the traffic, also equipped with data transmission systems to the centre.

The mobile laboratories (2) which monitor the quality of the air, are mobile stations which carry the sensors described above. The mobility of these stations is such as to cover more than one area and to collect data in those areas where the fixed stations are not installed.

The Operations Centre (7) collects, stores, manages and processes the data coming from the measurement networks described above: this centre provides also for the interrogation of all the stations connected to the network, retrieving their data and archiving it in suitable data bases. Some of these data bases provide the input data for the extrapolation procedure described earlier, which is then displayed at a suitable graphic workstation within the centre itself.
The data and forecast distribution system consists of a communications interface present at the operations centre which delivers the main data collected by the operations centre, to external users such as Local or Central Authorities, providing also data to the population on pollution through the best suited mass media.

## Claims

1. Integrated system for the detection and management of urban pollution and related procedures, consisting of:
fixed network for atmospheric pollutants (1);-weather network (4); - Traffic detector network (3); -Mobile laboratories for air quality control (2); -Operations Centre for storage, retrieval, management and processing of the data coming from the above mentioned measurement networks (7); -data and forecast distribution system (5) (6).

2. Integrated system to detect and manage urban pollution and related procedures, where the term integrated signifies that the procedure requires all components of the system in order to operate at full specification.

3. Integrated system for the management and detection of urban pollution and related procedures as per claims 1 and 2, where the fixed measurement network (1) for air pollutants, consists of a set of stations equipped with sensors to detect the main air pollutants and is provided with communications lines to send data to the operations centre.

4. Integrated system for urban pollution detection and management and related system as per claims 1, 2, 3, where the weather network (4) consists of a set of stations which contain the main weather sensors, also connected to the Operations Centre for data transmission.

5. Integrated system for the detection and management of urban pollution and related system as per claims 1,2,3,4, also including a network for automatic traffic detection (20), consisting of
- fixed positions, within which there are sensors to measure traffic rate, also capable of transmitting data to the Centre;
- laboratories to monitor air quality, consisting of mobile stations containing the instruments described at the previous points;
- operations centre to retrieve, store and manage the data coming from the measurement networks;
- data and forecast diffusion system consisting of a communications interface, present in the Operations Centre which sends the main information to external users following collection and processing>
